# FASCICULE DE BREVET EUROPEEN

(11) **EP 0 437 121 B1**
(45) Date de publication et mention de la délivrance du brevet: **20.08.1997**
(21) Numéro de dépôt: 90403150.7
(22) Date de dépôt: 07.11.1990
(51) Int. Cl.: A61F 2/02, A61M 25/00, B29C 65/02, B29C 71/04

(54) **Filtre-cathéter**
Filter-Katheter-Kombination
Filter-catheter combination

(30) Priorité: 13.12.1989 FR 8917201
(43) Date de publication de la demande: 17.07.1991
(60) Demande divisionnaire: 97200128.3
(73) Titulaire: Lefebvre, Jean-Marie, 59800 Lille (FR)
(72) Inventeur: Lefebvre, Jean-Marie, 59800 Lille (FR)
(74) Mandataire: Bruin, Cornelis Willem

(56) Documents cités:
- DE-A- 2 916 097
- FR-A- 2 227 022
- FR-A- 2 330 518
- FR-A- 2 343 488
- FR-A- 2 580 504
- FR-A- 2 606 642
- GB-A- 2 017 499
- GB-A- 2 054 385
- US-A- 3 866 599
- US-A- 4 085 185
- US-A- 4 154 242
- US-A- 4 608 965
- US-A- 4 636 272
- US-A- 4 801 297
- US-A- 4 832 055

## Description

La présente invention concerne un filtre pour l'interruption partielle et au moins temporaire d'une veine, comme décrit dans le préambule de la revendication 1.

Un tel filtre est connu, notamment par FR-A-2 606 642. On a constaté que la présence d'un tel filtre-cathéter dans une veine pouvait occasionner l'apparition de caillots sanguins, localisés à proximité immédiate dudit filtre.

L'objet de l'invention est de proposer un filtre-cathéter du type précité qui paillie à l'inconvénient constaté.

Ce but est atteint par un filtre pour l'interruption partielle et au moins temporatire d'une veine selon l'invention, comme caracterisé dans la revendication 1.

Le polytétrafluoréthylène propre est déja très peu thrombogénique. Pour obtenir la solidarisation de la canalisation et du conduit intérieur par thermosoudage, le filtre-cathéter selon l'invention ne comporte vers son extremité distale aucun autre matériau que le polytétra-fluoréthylène en contact avec le flux sanguin. L'organe radio-opaque étant un anneau dans un matériau radio-opaque, par exemple l'or, qui est inclus entre la canalisation et le conduit intérieur et fixé à ceux-ci lors du thermosoudage, cet élément radio-opaque n'est pas apparent et ne peut être source ni d'accrochage avec la paroi veineuse ni de formation éventuelle de caillots sanguins.

Ainsi l'invention fournit un filtre-cathéter qui ne provoque pas la formation de caillots sanguins.

Il est à noter que l'utilisation de la polytétrafluoréthylène pour un cathéter est connue en soi, par exemple du document GB-A-2 017 499. L'utilisation d'un anneau dans un matériau radio-opaque, est aussi connue en soi, par exemple du document FR-A-2 580 504.

D'autres caractéristiques et avantages ressortiront plus clairement de la description qui va être faite du mode préféré de réalisation du filtre-cathéter à usage temporaire, illustré par le dessin annexé dans lequel:
La figure 1 est une vue schématique en coupe du filtre au repos
La figure 2 est une vue schématique en coupe du filtre déployé
La figure 3 est une vue en coupe transversale du filtre selon l'axe D de la figure 2
La figure 4 est une vue schématique en coupe de l'extrémité distale d'un filtre
La figure 5 est une vue schématique de côté du dispositif de maintien du filtre-cathéter pendant le traitement de thermofixation.

Le filtre-cathéter 1 de l'invention se compose essentiellement d'une canalisation 2 creuse et d'un conduit creux 3 qui est placé coaxialement à l'intérieur de la canalisation 2. Ces deux éléments, canalisation 2 et conduit 3 , sont en polytétrafluoréthylène.

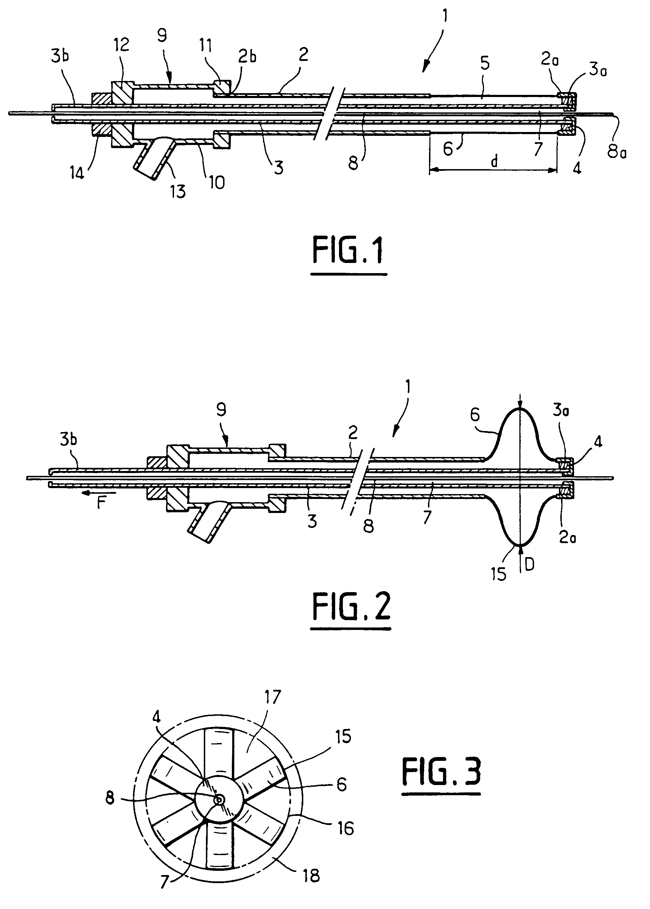

Dans le présent texte les termes "proximal" et "distal" sont employés en prenant pour référence la partie du corps du patient par où le filtre est introduit. Ainsi l'extrémité distale du filtre, représentée à droite sur les figures 1 et 2, est l'extrémité la plus éloignée de cette partie du corps, et l'extrémité proximale la plus rapprochée.

Les extrémités distales respectivement 2a de la canalisation 2 et 3a du conduit 3 sont thermosoudées ensemble et autour d'un anneau métallique 4, en acier inoxydable, platine ou or. Cet anneau 4 est dans un matériau radio-opaque, détectable lors du positionnement du filtre.

Comme cela ressort clairement des figures 1 et 2, l'anneau 4 est placé entre la canalisation 2 et l'extrémité distale 3a du conduit 3, l'extrémité distale 2a de la canalisation étant repliée à l'intérieur du conduit 3 et recouvrant l'extrémité distale 3a dudit conduit. Le thermosoudage réalise l'assemblage de ces éléments pour former l'extrémité 15 du filtre-cathéter.

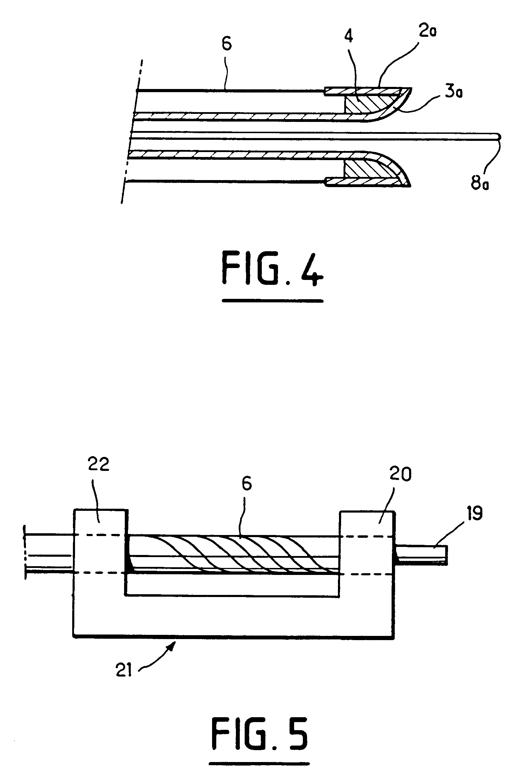

Selon un autre mode de réalisation illustré à la figure 4, le thermosoudage intervient alors que l'extrémité distale 3a du conduit intérieur 3 déborde au-delà de l'anneau métallique 4 et s'évase vers l'extérieur venant au contact de l'extrémité distale 2a de la canalisation 2.

En amont de son extrémité distale 2a, la canalisation 2 comporte six entailles 5, pratiquées dans le sens longitudinal sur une longueur d de 30 mm; elles sont régulièrement réparties sur la périphérie de la canalisation. Ces entailles 5 délimitent six bandes 6 identiques, chacune d'elles occupe un espace angulaire de 60° de la canalisation 2. Pour une canalisation 2 de 3 mm de diamètre, chaque bande 6 a une largeur d'environ 1,5 mm.

Le conduit 3 est creux et comporte un canal central 7, dans lequel peut librement coulisser une tige métallique 8 servant de tige de guidage lors de l'introduction du filtre-cathéter.

Vers l'extrémité proximale, mais à l'extérieur du corps du patient, la canalisation 2 est fixée sur un dispositif 9 de maintien et d'alimentation. Ce dispositif 9 comporte une tubulure cylindrique 1D et des bagues 11 et 12 d'extrémité. La première bague 11 annulaire raccorde l'extrémité proximale 2b de la canalisation 2 à la partie avant de la tubulure 10. La seconde bague 12 entoure le conduit 3 et ferme la partie arrière de la tubulure 10. Un coude 13 débouche en oblique dans le corps de la tubulure 10.

L'extrémité proximale 3b du conduit 3 va au-delà de la tubulure 10. La bague 12 enserre cette extrémité distale 3b de façon étanche ; toutefois le conduit 3 peut coulisser longitudinalement dans la bague 12 par rapport à la tubulure 10 et donc à la canalisation 2.

Une bague de blocage 14 est apte à assurer le blocage du conduit 3 contre la bague 12.

La tubulure 10 est fixée de manière connue à proximité du patient sur un bâti supportant deux systèmes d'alimentation de produits à injecter, débouchant pour le premier dans l'extrémité proximale 3b du conduit et pour le second dans le coude 13 de la tubulure 10.

La mise en oeuvre du filtre-cathéter se déroule dans les conditions suivantes.

Dans une première phase, la tige de guidage 8 est introduite par voie percutanée dans la veine adéquate, jusqu'à ce que son extrémité 8a distale se trouve en position dans la veine cave inférieure du patient.

Dans une deuxième phase, on fait coulisser depuis l'extérieur du corps du patient le filtre-cathéter 1 le long de la tige de guidage 8 jusqu'à ce que l'anneau 4 se trouve en position dans la veine cave, là où le filtre doit être implanté.

Pendant le déroulement de cette deuxième phase, les bandes 6 sont au repos, c'est-à-dire qu'elles sont dans le prolongement de la canalisation 2.

Dans une troisième phase illustrée par la figure 2 et correspondant au déploiement du filtre, la bague de blocage 14 est desserrée en sorte de permettre au conduit 3 de coulisser dans la bague 12. Puis on déplace avec précaution l'extrémité proximale 3b du conduit 3 vers l'arrière dans le sens de la flèche F. Dans cette phase, tout le conduit 3 se déplace ainsi que l'anneau 4, solidaire de l'extrémité distale 3a du conduit. Etant donné que la tubulure 10 et donc la canalisation 2 sont maintenues en position, le recul de l'anneau 4 dans le sens de la flèche F ne peut se produire que grâce à la présence des bandes 6 flexibles. Chaque bande 6 se plie individuellement, créant de ce fait une déformation transversale homogène de la canalisation dans la zone des entailles 5. Le déplacement et la longueur des bandes 6 sont choisis de telle sorte que, en position de déploiement, les parties médianes 15 des bandes 6, correspondant au plan D de courbure, prennent appui sur la paroi interne 16 de la veine. Une fois que le filtre est correctement déployé, la bague de blocage 14 est resserrée en sorte d'empêcher tout déplacement du conduit 3 par rapport à la canalisation 2 et donc de maintenir les bandes 6 dans leur état de déformation transversale.

Comme cela apparaît clairement sur la figure 3, qui est une coupe selon le plan D, les six bandes 6 sont déployées transversalement par rapport à la canalisation 2 et obturent partiellement l'intérieur 17 de la veine 18. Ainsi les caillots sanguins se déplaçant à l'intérieur 17 de la veine 18 sont arrêtés par les bandes 6 tandis que le sang peut circuler librement.

Préalablement à l'utilisation du filtre-cathéter, on a fait subir à celui-ci un traitement thermique de thermofixation. Ce traitement a consisté à donner un choc thermique à la partie distale du filtre alors que les bandes flexibles 6 sont dans leur état déployé, comme illustré à la figure 2. La durée de ce traitement et la température sont déterminées en sorte que la forme prise par les bandes soit en quelque sorte mémorisée dans la structure polymérique du polytétrafluoréthylène. Ce traitement augmente la résistance à la déformation des bandes 6 et évite l'affaissement du filtre à l'intérieur de la veine 18 et le risque de thrombogénèse dû aux turbulences créées dans le flux sanguin par l'hétérogénéité de disposition des bandes 6 dans la veine.

Selon un autre mode de réalisation du traitement de thermofixation illustré à la figure 5, le traitement thermique est appliqué sur la partie distale du filtre-cathéter alors que les bandes flexibles 6 ont une forme torsadée. Pour cela, on emmanche dans le conduit intérieur 3 un mandrin 19 de manière à rigidifier la partie distale du filtre-cathéter; on bloque en position l'extrémité distale 2a de la canalisation 2 dans la branche avant 2D d'un support de maintien 21, on fait subir à la canalisation 2 une rotation sur elle-même, qui confère aux bandes flexibles 6 la forme torsadée illustrée sur la figure 5, puis on bloque en position la canalisation 2 en amont desdites bandes flexibles 6 dans la branche arrière 22 du support de maintien 21. Le support 21 et la partie distale du filtre-cathéter sont placés dans un four de cuisson par exemple par rayonnement micro-onde pour y subir le traitement thermique, pendant 4 à 5 minutes. On remarque que lors de l'utilisation du filtre, les bandes flexibles 6 ont tendance à prendre la forme en hélice conférée lors du traitement thermique. Bien sûr sur le support de maintien 21, les bandes 6 peuvent être dans la position repos comme représenté sur la figure 5 ou dans l'état déployé.

Un produit de traitement thérapeutique peut être injecté en amont du filtre à travers le conduit 3, à partir de l'extrémité proximale 3b de celui-ci. Il s'agit d'un anticoagulant du type héparine ou d'un fibrinolytique. Le conduit 3 peut comporter des orifices, par exemple disposés en spirale, dans la zone faisant face aux bandes 6. Ainsi le produit de traitement thérapeutique introduit dans le conduit 3 peut être réparti à la fois en amont et au niveau du filtre lui-même.

Le même produit, ou éventuellement un autre produit de traitement thérapeutique peut être injecté dans la zone immédiatement en aval du filtre à travers la canalisation 2, à partir du coude 13 de la tubulure 10.

Une fois le traitement jugé satisfaisant, le retrait du filtre s'effectue de la manière suivante. La bague de blocage 14 est desserrée et l'extrémité proximale 3b du conduit 3 est repoussée dans le sens inverse de la flèche F jusqu'à ce que les languettes 6 retrouvent leur position de repos, rectilignes et parallèles entre elles. Puis, la bague 14 est de nouveau resserrée, et l'ensemble conduit 3 et canalisation 2 est retiré de la veine.

Les essais réalisés à l'aide du filtre-cathéter selon l'invention ont permis de constater l'absence de toute formation de caillots sanguins du fait de la présence du filtre dans la veine.

La présente invention n'est pas limitée au mode de réalisation qui a été décrit à titre d'exemple non exhaustif, mais en couvre toutes les variantes dans le cadre des revendications suivantes. En particulier la tige de guidage 8 peut être creuse et permettre l'injection de produit de traitement en aval du filtre voire même bien au-delà de l'extrémité distale du filtre cathéter 1.

## Revendications

1. Filtre pour l'interruption partielle et au moins temporaire d'une veine qui comporte:
a. une canalisation (2) présentant vers son extrémité distale des entailles longitudinales réparties symétriquement sur sa périphérie et délimitant des bandes (6) flexibles;
b. et un conduit (3) intérieur à ladite canalisation, la canalisation (2) et le conduit (3) étant solidaires par leurs extrémités distales (2a, 3a), l'extrémité distale du filtre étant équipée d'un organe radio-opaque;
caractérisé en ce que la canalisation (2) et le conduit intérieur sont en polytétrafluoréthylène, les extrémités distales (2a, 3a) de la canalisation (2) et du conduit (3) étant thermosoudées, et en ce que l'organe radio-opaque comporte un anneau (4), dans un matériau radio-opaque qui est inclus entre les extrémités distales (2a, 3a) de la canalisation (2) et du conduit (3) et fixé à celles-ci lors du thermosoudage.

2. Filtre selon la revendication 1, caractérisé en ce que l'extrémité distale (2a) de la canalisation est repliée vers l'intérieur du conduit (3) et recouvre d'une part l'anneau (4) et d'autre part l'extrémité distale (3a) du conduit (3).

3. Filtre selon la revendication 1 caractérisé en ce que l'extrémité distale (3a) du conduit intérieur (3) s'évase vers l'extérieur au-delà de l'anneau métallique (4).

4. Filtre selon la revendication 1 caractérisé en ce que le conduit (3) comporte des orifices dans la zone située en regard des bandes (6).

5. Filtre selon la revendication 1 caractérisé en ce que , la partie distale dudit filtre ayant subi un traitement de thermofixation , les bandes flexibles (6) ont une résistance à la déformation améliorée.

6. Filtre selon la revendication 5 caractérisé en ce que , le traitement de thermofixation ayant été réalisé alors que les bandes (6) étaient à l'état déployé, le polytétrafluoréthylène constituant les bandes flexibles (6) a en mémoire ladite forme déployée.

7. Filtre selon l'une des revendication 5 ou 6 caractérisé en ce que, le traitement de thermofixation ayant été réalisé alors que les bandes (6) étaient tordues en hélice, le polytétrafluoréthylène constituant les bandes flexibles (6) a en mémoire ladite forme en hélice.

## Claims

1. Filter for the partial and at least temporary interruption of a vein which includes:
a. a pipe (2) having towards its distal end longitudinal scores distributed symmetrically around its periphery and defining flexible strips (6);
b. and a conduit (3) within the pipe, the pipe (2) and the conduit (3) being firmly attached by their distal ends (2a, 3a), the distal end of the filter being provided with a radio-opaque organ;
characterised by the fact that the pipe (2) and the internal conduit are made of polytetrafluoroethylene, the distal ends (2a, 3a) of the pipe (2) and the conduit (3) being thermowelded and by the fact that the radio-opaque organ includes a ring (4), in a radio-opaque material which is contained between the distal ends (2a, 3a) of the pipe (2) and the conduit (3) and attached to these during the thermowelding.

2. Filter as described in claim 1, characterised by the fact that the distal end (2a) of the pipe is bent towards the inside of the conduit (3) and covers on the one hand the ring (4) and on the other hand the distal end (3a) of the conduit (3).

3. Filter as described in claim 1, characterised by the fact that the distal end (3a) of the internal conduit (3) is flared outwardly beyond the metal ring (4).

4. Filter as described in claim 1, characterised by the fact that the conduit (3) has orifices in the zone situated opposite the strips (6).

5. Filter as described in claim 1, characterised by the fact that, the distal part of the said filter having undergone a thermofixing treatment, the flexible strips (6) have an enhanced resistance to deformation.

6. Filter as described in claim 5, characterised by the fact that, the thermofixing treatment having been performed while the strips (6) were in the deployed state, the polytetrafluoroethylene forming the flexible strips (6) retains the memory of the said deployed shape.

7. Filter as described in one of claims 5 or 6, characterised by the fact that, the thermofixing treatment having been performed while the strips (6) were twisted in a helix, the polytetrafluoroethylene forming the flexible strips (6) retains the memory of the said helical shape.

## Patentansprüche

1. Filter zur teilweisen und zumindest vorübergehenden Unterbrechung eine Vene, welches aufweist:
a) einen Kanal (2), der zu seinem distalen Ende hin sich in der Längsrichtung erstreckende Einkerbungen hat, die auf seinem Umfang symmetrisch verteilt sind und biegsame Bänder (6) begrenzen;
b) und eine Leitung (3) im Innern des Kanals, wobei der Kanal (2) und die Leitung (3) über ihre distalen Enden (2a, 3a) verbunden sind und das distale Ende des Filters mit einem strahlungsundurchlässigen Element ausgestattet ist;
dadurch **gekennzeichnet**, daß der Kanal (2) und die innere Leitung aus Polytetrafluorethylen bestehen, wobei die distalen Enden (2a, 3a) des Kanals (2) und der Leitung (3) thermoverschweißt sind, und daß das Röntgenundurchlässige Element einen Ring (4) in einem Röntgenundurchlässigen Material aufweist, das zwischen den distalen Enden (2a, 3a) des Kanals (2) und der Leitung (3) enthalten ist und an ihnen beim Thermoverschweißen befestigt wird.

2. Filter nach Anspruch 1, dadurch gekennzeichnet, daß das distale Ende (2a) des Kanals zum Innern der Leitung (3) hin umgeschlagen ist und einerseits den Ring (4) und andererseits das distale Ende (3a) der Leitung (3) überdeckt.

3. Filter nach Anspruch 1, dadurch gekennzeichnet, daß das distale Ende (3a) der inneren Leitung (3) sich nach außen hin über den metallischen Ring (4) erweitert.

4. Filter nach Anspruch 1, dadurch gekennzeichnet, daß die Leitung (3) Öffnungen in dem Bereich gegenüber der Bänder (6) aufweist.

5. Filter nach Anspruch 1, dadurch gekennzeichnet, daß der distale Teil des Filters einer Thermofixierbehandlung ausgesetzt wurde und die biegsamen Bänder (6) einen verbesserten Verformungswiderstand haben.

6. Filter nach Anspruch 5, dadurch gekennzeichnet, daß nach Beendigung der Thermofixierbehandlung, während der die Bänder (6) im entfalteten Zustand waren, das die biegsamen Bänder (6) bildende Polytetrafluorethylen die entfaltete Form im Gedächtnis hat.

7. Filter nach einem der Ansprüche 5 oder 6, dadurch gekennzeichnet, daß nach Beendigung der Thermofixierbehandlung, während der die Bänder (6) zu einer Schraube verdreht waren, das die biegsamen Bänder (6) bildende Polytetrafluorethylen die Schraubenform im Gedächtnis hat.
